# EUROPEAN PATENT SPECIFICATION

(11) **EP 1 424 995 B1**
(45) Date of publication and mention of the grant of the patent: **14.07.2010**
(21) Application number: 02798023.4
(22) Date of filing: 10.09.2002
(51) Int. Cl.: A61K 9/00, A61K 38/16, A61D 1/02

(54) **A BIO-SECURITY SYSTEM**
BIOSICHERHEITSSYTEM
SYSTEME DE BIO-SECURITE

(30) Priority: 10.09.2001 US 317934 P
(43) Date of publication of application: 09.06.2004
(73) Proprietor: BIMEDA RESEARCH AND DEVELOPMENT LIMITED, DUBLIN 24 (IE)
(72) Inventor: MCHARDY, Nicholas, County Wicklow (IE); HALLAHAN, Stephen, Dublin 8, (IE)
(74) Representative: O'Brien, John Augustine
(86) International application number: PCT/IE2002/000131
(87) International publication number: WO 2003/022245

(56) References cited:
- EP-A- 0 076 068
- WO-A-98/26759
- GB-A- 2 273 655
- TWOMEY DP ET AL: "Protection against Staphylococcus aureus Mastitis in dairy cows using a Bismuth-based teat seal containing the bacteriocin, lacticin 3147." JOURNAL OF DAIRY SCIENCE, vol. 83, no. 9, September 2000 (2000-09), pages 1981-1988, XP002189812 ISSN: 0022-0302

## Description

The invention relates to a method for preventing contamination of a bovine teat during administration of a sealant intended to prevent mastitis.

Bovine mastitis is a severe, potentially fatal, inflammatory disease of the udder, caused by a broad range of infectious organisms, but most notably by various Gram positive bacteria of the genera *Staphylococcus* and *Streptococcus* and the Gram negative species, *Escherichia coli*. The infection usually enters the udder via the teat or streak canal. Mastitis is treated by a variety of antibiotic cerates, infused into the udder via the teat or streak canal. In severe cases, high doses of antibiotic are also given by injection. A high proportion of mastitic infections are contracted during the "dry" period, which precedes calving. The infection may later become clinically significant either during the dry period, or after calving when lactation has resumed.

It is common practice to seek to reduce the incidence of clinical mastitis by infusing high doses of antibiotic formulated as a "slow release" cerate into the udder at drying off. This eliminates infections that are already present in the udder and it also gives lasting protection against new infections that may be contracted during the dry period. The ethical and clinical justification for such treatment is questionable, however, as only a minority of cows are likely to be infected at drying-off, and only a minority are likely to contract new infections during the dry period. However, until recently, there has been no adequately effective alternative to such blanket antibiotic dry cow treatments.

One product, Teat Seal (Trade Mark of Cross Vetpharm), provides an effective, antibiotic-free, alternative. The Teat Seal is described in detail in PCT/IE97/00085, published as WO 98/26759 A. The sealant comprises a viscous oil-based cerate containing a high proportion of a heavy metal salt, bismuth subnitrate, which is infused into the teat at drying-off. It remains in the teat for the entire dry period. It prevents infection entering the udder via the teat or streak canal through a combination of its viscosity, density and adhesiveness. However, since this product contains no antibiotic, it is essential that the surface of the teat is thoroughly sanitised and that the sealant injector itself is entirely sterile, to avoid the accidental introduction of mastitis-causing organisms into the teat during infusion of the product. Safe application of the product can be achieved consistently by an experienced operator with access to suitable cleansing materials, but such conditions do not always exist on farms.

Incorporation into the sealant product of a non-antibiotic but none-the-less antibacterial substance such as a bacteriocin can greatly reduce the incidence of infection even in the face of deliberately severe bacterial "challenge" (PCT/IE96/00022), published as WO 96/32482 A.

However, whilst undoubtedly effective, this procedure has significant shortcomings. Among these are that it requires a large amount of bacteriocin such as lacticin (between 10,000 and 100,000 AU), which may be irritant to the udder. In addition, the sealant must be specially formulated to permit the release of the lacticin from the sealant at the required rate while not interfering with the normal functioning of the sealant. This is extremely difficult to achieve, particularly on a commercial manufacturing scale, because the precise physical consistency of the sealant is essential to its correct functioning. Formulation of a sealant to perform two distinct functions is therefore likely to be a compromise, such that neither is performed completely adequately. EP 0076068 A and CrB 2273655 A disclose further compositions comprising a sealant and an antibacterial compound.

There is therefore a need for a system which prevents or reduces the risk of accidentally introducing mastitis-causing organisms into the teat when administering a sealant or other products for treating or preventing mastitis.

### Statements of invention

According to the invention there is provided an injector for preventing contamination of a non-human teat during administration of a sealant having a barrel for storage of a sealant, a nozzle extending from the barrel, and a sterilising agent provided on the outside surface of the nozzle so that in use the sterilising agent is delivered into the teat in advance of the sealant such that the sealant is deliverable into the teat through the sterilising agent.

Preferably the injector has a removable cap for the nozzle, the sterilising agent being provided between the cap and the nozzle.

Preferably the sterilising agent comprises a bacteriocin, most preferably the bacteriocin is Lacticin 3147.

Ideally when the sterilising agent is provided between the cap and the nozzle the bacteriocin is present in an amount of from 100 to 100,000AU.

Preferably the sterilising agent is selected from any one or more of an alcohol, propionic acid, benzyl alcohol, methyl paraben, propyl paraben, sorbic acid, potassium sorbate, benzoic acid, sodium benzoate, bronopol, chlorhexidine, cetrimide, butyl hydroxyanisole, butyl hydroxytoluene or salts thereof, most preferably an alcohol.

In one embodiment of the invention the sterilising agent is in the form of a water miscible gel.

In another embodiment of the invention the sterilising agent is in the form of an oil based gel.

In a further embodiment of the invention the sterilising agent is in the form of an oil based paste.

In one embodiment of the invention the sterilising agent is provided in the barrel downstream of the sealant. Preferably there is a frangible barrier between the sealant and the sterilising agent.

In another embodiment of the invention the sterilising agent is provided in the nozzle. In this case the sterilising agent may be in the form of a gel which is fluid when hot. The gel is loaded into an injector in fluid form so that the gel uniformly fills the nozzle and rapidly sets as it cools. The sealant is loaded subsequently into the injector. This ensures that the sterilising agent is extruded from the injector ahead of the sealant material.

Preferably the injector comprises a removable cap for the nozzle and the sterilising agent is provided between the nozzle and the removable protective cap.

Preferably the volume of the sterilising agent used for a single teat seal injector is between 0.01ml and 10ml, most preferably approximately between 0.05ml and 2.0ml.

Preferably the sterilising agent comprises a bacteriocin, most preferably Lacticin 3147. Preferably the bacteriocin is present in an amount of from 100 to 100,000AU.

Preferably the sterilising agent is selected from any one or more of an alcohol, propionic acid, benzyl alcohol, methyl paraben, propyl paraben, sorbic acid, potassium sorbate, benzoic acid, sodium benzoate, bronopol, chlorhexidine, cetrimide, butyl hydroxyanisole, butyl hydroxytoluene or salts thereof, most preferably the antiseptic is an alcohol.

The sterilising agent may be in the form of a water miscible gel, an oil based gel or an oil based paste.

### Brief description of Drawings

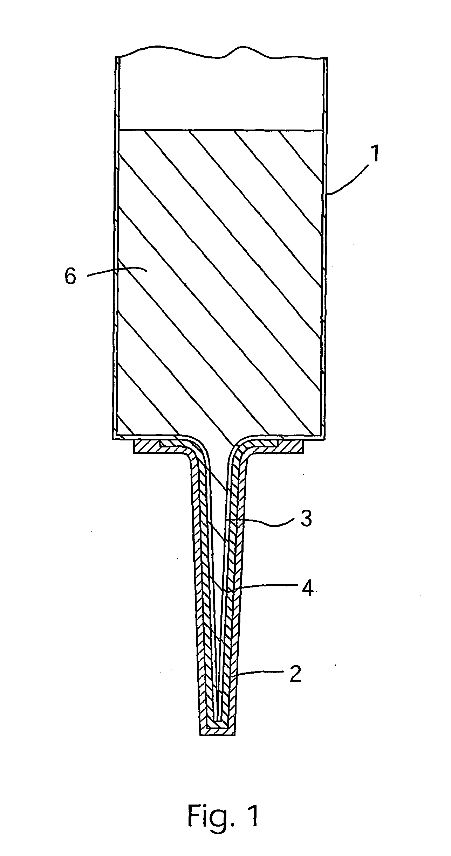

The invention will be more clearly understood from the following description thereof given by way of example only with reference to the accompanying drawings, in which:-
Fig. 1 is a schematic representation of the front portion of a sealant injector with a protective cap in place protecting the injector nozzle of the invention;
Fig. 2 is a schematic representation of the sealant injector of Fig. 1 with the protective cap removed;
Fig. 3 is a schematic representation of the sealant injector of Figs. 1 and 2 on insertion into the teat or streak canal;

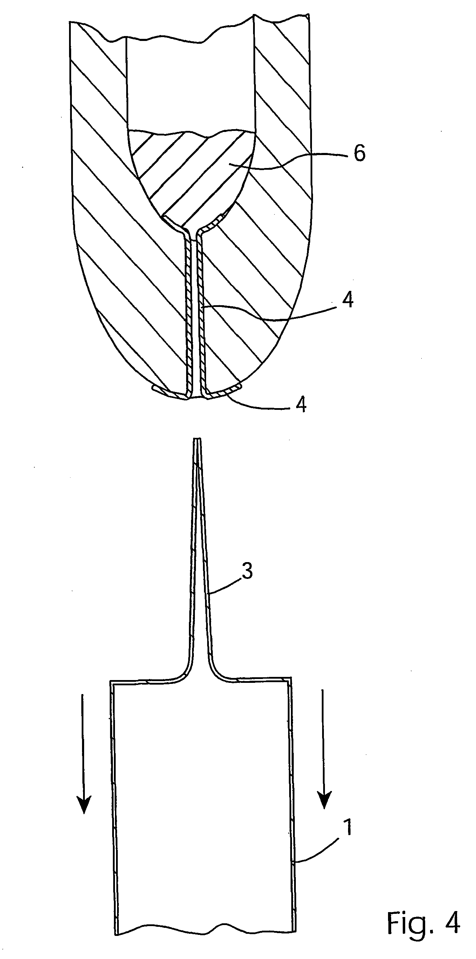

Fig. 4 is a schematic representation of the sealant injector of Fig. 1 and 2 showing removal of the applicator nozzle from the teat;
Fig. 5 is a schematic representation of another injector; and
Fig. 6 is a schematic representation of a further injector.

### Detailed Description

The invention provides a system which substantially prevents contamination of a teat, in particular a bovine teat, during administration of a sealant product in a mastitis preventative treatment. A sterilising agent is deliverable into the teat or streak canal directly in advance of a mastitis preventative treatment to reduce the risk of introducing mastitis-causing organisms when treating the teat. The invention may be used with other mastitis preventing products, however the invention is particularly important when using a product that does not itself contain an anti-infective substance.

The method of the invention directly delivers a sterilising agent into the teat or streak canal ahead of the sealant thereby ensuring that accidentally introduced organisms are killed and do not cause mastitis. The sterilising agent is carried into the teat ahead of the sealant such that the sealant is delivered to the teat through the sterilising agent.

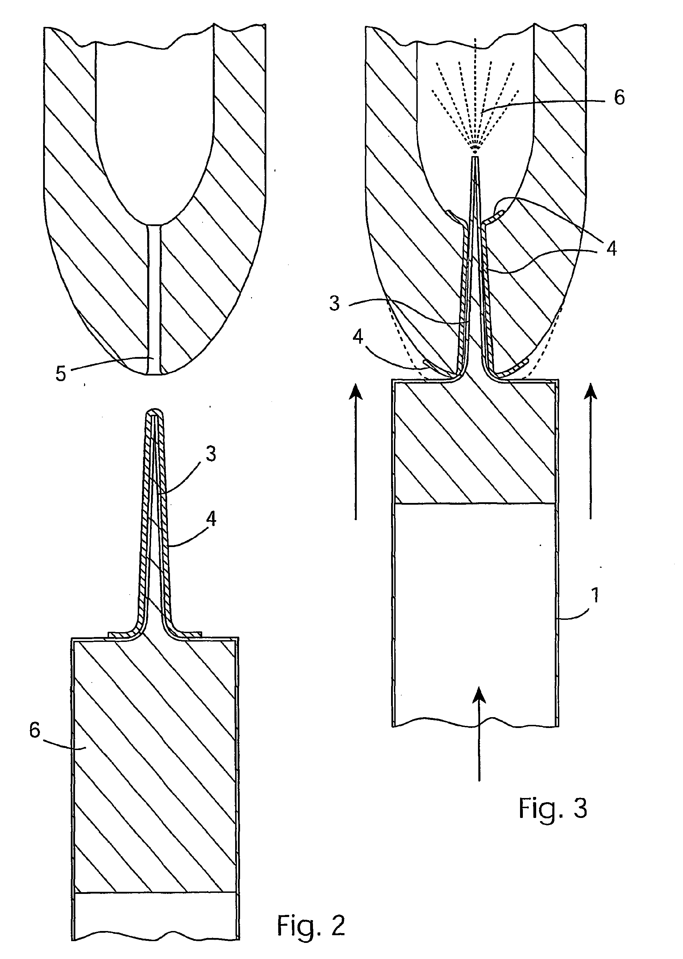

Referring to the drawings there is illustrated a sealant injector 1 having a nozzle 3. A removable sealing or protective cap 2 covers the nozzle 3 of the injector 1 during storage and transportation for the purpose of maintaining sterility of the nozzle 3 and of the contents of the sealant injector 1. A sterilising agent 4 is provided between the protective cap 2 and the nozzle 3. When the protective cap 2 is removed, immediately prior to administration of the sealant, a sufficient amount of the sterilising agent 4 remains coated to the outside of the nozzle 3 as shown in particular in Fig. 2, rendering and maintaining the nozzle sterile. When the nozzle 3 is inserted into the teat or streak canal 5, it carries sufficient of the sterilising agent 4 into the teat canal 5 to form an anti-infective "barrier" ahead of infusion of the sealant 6. On insertion of the injector nozzle into the teat canal some of the sterilising agent is also left on the outer skin of the teat canal orifice, thereby killing any bacteria which might otherwise be carried into the teat as the nozzle 3 is inserted.

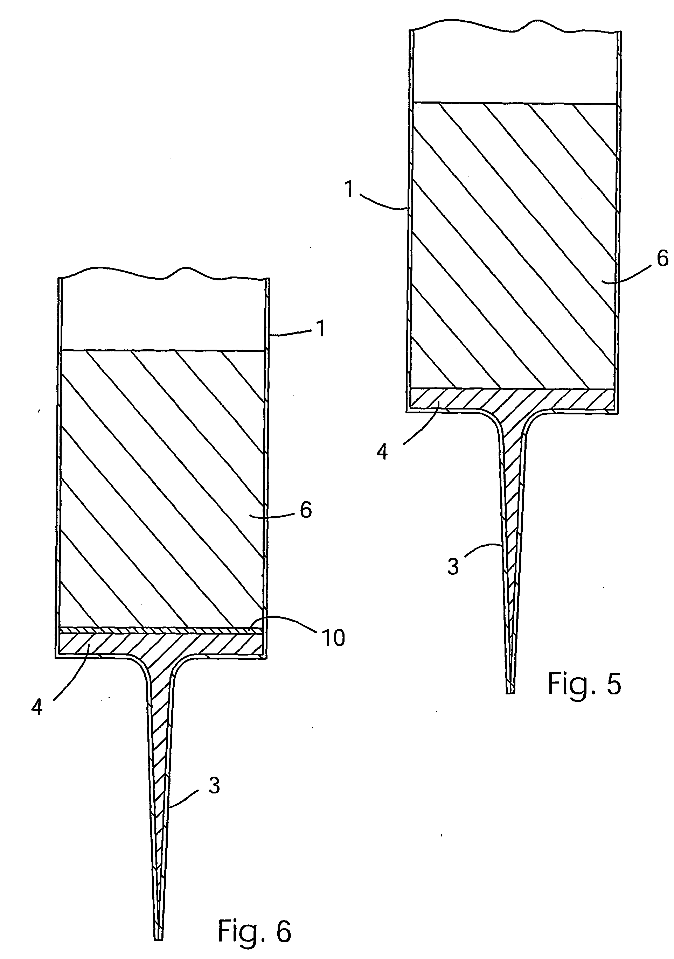

Alternatively the sterilising agent 4 may be loaded into the sealant injector 1 itself as shown in particular in Figs. 5 and 6 such that it is delivered into the teat canal ahead of the sealant 6 itself during normal infusion of the sealant. The sterilising agent 4 is placed in the barrel of the injector 1 ahead of the sealant product 6 such that it is extruded first, through the injector nozzle 3.

The sterilising agent may be a water-based pharmaceutical gel which is immiscible with the oil-based gel of the sealant product itself, such that the physical and pharmaceutical stability of both materials is not compromised by contact or admixture.

Alternatively a burstable/frangible membrane 10 or similar separating device may be located in the barrel of the injector 1 to provide both a physical and chemical separation of the sealant and the sterilising agent.

Any suitable sterilising agent which is compatible with the sealant and its application may be used. The purpose of the agent and the quantity utilised is to sanitise, clean and/or sterilise the teat or streak canal in advance of a sealant.

The viscosity of the sterilising agent should be such that, if it is placed within the barrel of the injector, it can be dispensed easily through the nozzle. At the same time, it is sufficiently viscous as not to leak from the injector during storage and transport. When the sterilising agent is placed within the protective cap which covers the nozzle, its consistency is such that an adequate amount adheres to the nozzle and is carried into the teat canal when the nozzle is inserted. The viscosity also allows for a small amount to be deposited and retained on the skin surrounding the teat orifice as the nozzle is inserted.

The sterilising agent used in the invention may comprise an anti-bacterial such as a bacteriocin. Bacteriocins are proteins secreted by bacteria that kill and sometimes lyse related bacteria. An example of a bacteriocin is Lacticin 3147 which is produced by the GRAS (generally received as safe) organism *Lactococcus lactis* DPC 3147. Lacticin 3147 is the subject of PCT patent application PCT/IE96/00022 published as WO96/32482. Other suitable bacteriocins include Nisin or Lysostatin.

Lacticin 3147 inhibits a wide range of anti-bacterial species including all of those most likely to cause mastitis such as *Streptococci* and *Staphylococci*. Lacticin 3147 is effective at physiological pH. It is a membrane-active pre-forming complex which exhibits a bactericidal mode of action causing cell death but not cell lysis. Lacticin 3147 is particularly suitable because it is temperature and pH stable, which are important factors during the manufacture and long term stability of the product. It also exerts maximal antibacterial effect at physiological pH levels, unlike some other bacteriocins.

The antiseptic properties of the sterilising agent may be achieved by the bacteriocin alone, a chemical antiseptic alone or a combination of a bacteriocin and an antiseptic chemical. Various chemical sterilising agents/antiseptics may be used, such as alcohol, propionic acid and salts thereof, benzyl alcohol, methyl paraben and salts thereof, propyl paraben and salts thereof, sorbic acid, potassium sorbate, benzoic acid, sodium benzoate, bronopol, chlorhexidine, cetrimide, butyl hydroxyanisole or butyl hydroxytoluene. The principal purpose of the sterilising agent/chemical antiseptic is to kill Gram negative bacteria such as *Escherischia coli*, against which the bacteriocins are generally not as effective. Bacteriocins have marginal Gram negative activity. The antiseptics are also effective against Gram positive bacteria, so they may supplement or replace the bacteriocin in the invention.

The sterilising agent is intended only to kill bacteria which may have accidentally been introduced into the teat canal during the infusion of a sealant material, so it is required to be effective for only a short time and to present only a small amount of the anti-infective substance. An amount of bacteriocin typically between 100 and 100,000 AU (antibacterial units) may be included in each sealant injector device.

Various sterilising agents may be used, either alone or together with either a bacteriocin or a chemical antiseptic, or both of these. The sterilising agent may also comprise an antibiotic or antibacterial pharmaceutical substance. Suitable antibiotic or antibacterial pharmaceutical substances may include any one or more of a penicillin, a tetracycline, an aminoglycoside or similar broad spectrum antibiotic.

The water miscible gel, oil-based gel or oil based paste may comprise other components such as a thickening agent and/or other excipients. Suitable thickening agents for the water miscible gel include any one or more of methylcellulose 400 (or other soluble grade), alginic acid, sodium alginate or pre-gelatinised starch (1500). Suitable excipients include any one or more of polyvinylpyrrolidone, propylene glycol, glycerine, Tween 80 and water for injection. Suitable thickening agents for the oil-based gel or oil based paste include any one or more of aluminium stearate (ALUGEL), anhydrous silicon dioxide (Aerosil) or white soft paraffin (petroleum jelly). Other suitable excipients include any one or more of corn oil or other vegetable oil, liquid paraffin, hydrogenated oil or polyethyleneglycol (various grades). The water miscible or oil based gel or oil based paste may also include any suitable colouring agent which is pharmaceutically acceptable.

The volume of sterilising agent used with each sealant injector is typically between 0.01ml and 10ml. When the sterilising agent is placed in the protective cap of the injector, the volume will typically be between from 0.05ml and 2.0ml. If the sterilising agent is placed within the barrel of the injector the volume is typically between from 0.2ml and 5.0ml.

The current sealant injector device and its protective sealing cap, and the methodology of filling it during manufacture, are suitable, without modification, to accommodate the sterilising agent at least in some aspects. However, the barrel of the injector syringe, the sealing/protective cap and the filling procedure may each be modified in some aspects. For example, the profile of the protective cap may be changed to accommodate either the volume or the consistency of the formulation, and a suitable two-station filler may be used to place the sealant gel and the sterilising agent separately, into the barrel and or the protective cap. In the event that the sterilising agent is located in the cap, a handling device to permit filling of the cap with gel and its subsequent replacement over the nozzle onto the delivery syringe may be used.

The sterilising agent used in the invention is preferably intended for use with a single use sealant injector device. Such injector devices may be used for administering a sealant for the treatment and/or prophylaxis of mastitis in cattle or sheep.

## Claims

1. An injector for preventing contamination of a non-human teat during administration of a sealant having a barrel for storage of a sealant, a nozzle emending from the barrel, and a sterilising agent provided on the outside surface of the nozzle so that in use the sterilising agent is delivered into the teat in advance of the sealant such that the sealant is deliverable into the teat through the sterilising agent.

2. An injector as claimed in claim 1 wherein the injector comprises a removable cap for the nozzle and the sterilising agent is provided between the nozzle and the removable protective cap.

3. An injector as claimed in claim 1 or 2 wherein the volume of the sterilising agent used for a single teat seal injector is between 0.01 ml and 10ml.

4. An injector as claimed in claim 3 wherein the volume of the sterilising agent used for a single teat seal injector is between 0.05ml and 2.0ml.

5. An injector as claimed in any of claims 1 to 4 wherein the sterilising agent comprises a bacteriocin.

6. An injector as claimed in claim 5 wherein the bacteriocin is Lacticin 3147.

7. An injector as claimed in claim 5 or 6 wherein the bacteriocin is present in an amount of from 100 to 100,000AU.

8. An injector as claimed in any of claims 1 to 4 wherein the sterilising agent is selected from any one or more of an alcohol, propionic acid, benzyl alcohol, methyl paraben, propyl paraben, sorbic acid, potassium sorbate, benzoic acid, sodium benzoate, bronopol, chlorhexidine, cetrimide, butyl hydroxyanisole, butyl hydroxytoluene or salts thereof.

9. An injector as claimed in claim 8 wherein the sterilising agent is an alcohol.

10. An injector as claimed in any of claims 1 to 9 wherein the sterilising agent is in the form of a water miscible gel.

11. An injector as claimed in any of claims 1 to 9 wherein the sterilising agent is in the form of an oil based gel.

12. An injector as claimed in any of claims 1 to 9 wherein the sterilising agent is in the form of an oil based paste.

## Patentansprüche

1. Injektor zum Verhindern der Verunreinigung einer nichtmenschlichen Zitze während Verabreichung einer Dichtungsmasse, der eine Trommel zur Lagern einer Dichtungsmasse, eine sich aus der Trommel erstreckende Düse, und ein Sterilisierungsmittel umfasst, das auf der Außenfläche der Düse vorgesehen ist, so dass bei Gebrauch das Sterilisierungsmittel in die Zitze vor der Dichtungsmasse eingebracht wird, damit die Dichtungsmasse durch das Sterilisierungsmittel in die Zitze eingebracht werden kann.

2. Injektor nach Anspruch 1, wobei der Injektor eine entfernbare Kappe für die Düse aufweist und das Sterilisierungsmittel zwischen der Düse und der entfernbaren Schutzkappe vorgesehen ist.

3. Injektor nach Anspruch 1 oder 2, bei dem das Volumen des Sterilisierungsmittels, das für einen einzelnen Teat Seal-Injektor verwendet wird, zwischen 0,01 ml und 10 ml beträgt.

4. Injektor nach Anspruch 3, bei dem das Volumen des Sterilisierungsmittels, das für einen einzelnen Teat Seal-Injektor verwendet wird, zwischen 0,05 ml und 2,0 ml beträgt.

5. Injektor nach einem der Ansprüche 1 bis 4, bei dem das Sterilisierungsmittel ein Bakteriozin aufweist.

6. Injektor nach Anspruch 5, bei dem das Bakteriozin Lactizin 3147 ist.

7. Injektor nach Anspruch 5 oder 6, bei dem das Bakteriozin in einer Menge von 100 bis 100.000 AU vorliegt.

8. Injektor nach einem der Ansprüche 1 bis 4, bei dem das Sterilisierungsmittel von einem oder mehreren eines Alkohols, Propionsäure, Benzylalkohol, Methylparaben, Propylparaben, Sorbinsäure, Kaliumsorbat, Benzoesäure, Natriumbenzoat, Bronopol, Chlorhexidin, Cetrimid, Butylhydroxyanisol, Butylhydroxytoluol oder deren Salzen ausgewählt ist.

9. Injektor nach Anspruch 8, bei dem das Sterilisierungsmittel ein Alkohol ist.

10. Injektor nach einem der Ansprüche 1 bis 9, bei dem das Sterilisierungsmittel in Form eines wassermischbaren Gels vorliegt.

11. Injektor nach einem der Ansprüche 1 bis 9, bei dem das Sterilisierungsmittel in Form eines auf Öl basierenden Gels vorliegt.

12. Injektor nach einem der Ansprüche 1 bis 9, bei dem das Sterilisierungsmittel in Form einer auf Öl basierenden Paste vorliegt.

## Revendications

1. Injecteur permettant d'empêcher la contamination d'une tétine non humaine au cours de l'administration d'un produit de scellement ayant un cylindre destiné à stocker un produit de scellement, une buse s'étendant depuis le cylindre, et un agent de stérilisation mis en oeuvre sur la surface extérieure de la buse de sorte que, lors de l'utilisation, l'agent de stérilisation est administré dans la tétine avant le produit de scellement de telle manière que le produit de scellement est en mesure d'être administré dans la tétine au travers de l'agent de stérilisation.

2. Injecteur selon la revendication 1, dans lequel l'injecteur comporte un capuchon amovible pour la buse et l'agent de stérilisation est mis en oeuvre entre la buse et le capuchon de protection amovible.

3. Injecteur selon la revendication 1 ou la revendication 2, dans lequel le volume d'agent de stérilisation utilisé pour un injecteur de produit de scellement de tétine simple se situe entre 0,01 ml et 10 ml.

4. Injecteur selon la revendication 3, dans lequel le volume d'agent de stérilisation utilisé pour un injecteur de produit de scellement de tétine simple se situe entre 0,05 ml et 2,0 ml.

5. Injecteur selon l'une quelconque des revendications 1 à 4, dans lequel l'agent de stérilisation comporte une bactériocine.

6. Injecteur selon la revendication 5, dans lequel la bactériocine est le produit Lacticin 3147.

7. Injecteur selon la revendication 5 ou la revendication 6, dans lequel la bactériocine est présente dans une quantité allant de 100 à 100 000 UA.

8. Injecteur selon l'une quelconque des revendications 1 à 4, dans lequel l'agent de stérilisation est sélectionné parmi l'un quelconque ou plus entre un alcool, un acide propionique, un alcool benzylique, un méthylparaben, un propylparaben, un acide sorbique, un sorbate de potassium, un acide benzoïque, un benzoate de sodium, un bronopol, une chlorhexidine, un cétrimonium, un hydroxyanisole butylé, un hydroxytoluène butylé ou des sels de ceux-ci.

9. Injecteur selon la revendication 8, dans lequel l'agent de stérilisation est un alcool.

10. Injecteur selon l'une quelconque des revendications 1 à 9, dans lequel l'agent de stérilisation est sous le forme d'un gel miscible avec l'eau.

11. Injecteur selon l'une quelconque des revendications 1 à 9, dans lequel l'agent de stérilisation est sous le forme d'un gel à base d'huile.

12. Injecteur selon l'une quelconque des revendications 1 à 9, dans lequel l'agent de stérilisation est sous le forme d'une pâte à base d'huile.
